Europäisches Patentamt

European Patent Office

Office européen des brevets

(11) Veröffentlichungsnummer: **0 305 890 B1**

(12) **EUROPÄISCHE PATENTSCHRIFT**

(45) Veröffentlichungstag der Patentschrift: **13.10.93**

(21) Anmeldenummer: **88113831.7**

(22) Anmeldetag: **25.08.88**

(51) Int. Cl.5: **A61K 31/19**

Die Akte enthält technische Angaben, die nach dem Eingang der Anmeldung eingereicht wurden und die nicht in dieser Patentschrift enthalten sind.

(54) **Verwendung von Bezafibrat zur Behandlung von Diabetes.**

(30) Priorität: **01.09.87 DE 3729209**

(43) Veröffentlichungstag der Anmeldung:
**08.03.89 Patentblatt 89/10**

(45) Bekanntmachung des Hinweises auf die Patenterteilung:
**13.10.93 Patentblatt 93/41**

(84) Benannte Vertragsstaaten:
**AT BE CH DE ES FR GB GR IT LI LU NL SE**

(56) Entgegenhaltungen:

**DTSCH. MED. WSCHR., Band 107, Nr. 39, 1982, Seiten 1470-1473, Georg Thieme Verlag, Stuttgart, DE; E. RÜTH et al.: "Verbesserung der Diabeteseinstellung unter der Therapie mit Bezafibrat"**

**ARZNEIM. FORSCHUNG/DRUG RES., Band 34 (II), Nr. 9, 1984, Seiten 1038-1041; K.H. VOGELBERG et al.: "Der Somatostatin-Infusionstest zur Beurteilung der Glukose-Utilisation unter Bezafibrat-Medikation"**

**MEDWELT, Band 35, Nr. 11, 1984, Seiten 357-360; H. BRUNEDER et al.:**

**"Hyperlipoproteinämie und Diabetes mellitus"**

**MÜNCHEN MED. WSCHR., Band 124, Nr. 21, 1982, Seiten 535-537; H.U. JANKA et al.: "Kohlenhydratstoffwechsel unter Bezafibrat-Therapie"**

**DTSCH. MED. WSCHR., Band 106, Nr. 49, 1981, Seiten 1653-1656, Georg Thieme Verlag, Stuttgart, DE; H. BRUNEDER et al.: "Behandlung der Hyperlipoproteinämie bei Diabetikern"**

(73) Patentinhaber: **BOEHRINGER MANNHEIM GMBH**

**D-68298 Mannheim(DE)**

(72) Erfinder: **Beck, Walter, Dr. rer. nat. Andreas-Hoferstrasse 46 D-6800 Mannheim 51(DE)**

**Beschreibung**

Die vorliegende Erfindung betrifft die neuartige Verwendung von Bezafibrat (für die Herstellung von Arzneimitteln) zur Behandlung von Diabetes mellitus.

Bezafibrat - 2-[4-[2-(4-Chlorbenzamido)-ethyl]phenoxy]-2-methylpropionsäure - ist ein aus der DE-A-21 49 070 bekanntes, in großem Umfang zur Behandlung von Hyperlipidämien (Hypertriglyceridämien und Hyperchloresterinämien) eingesetztes Mittel. Da Hyperlipoproteinämien eine besonders häufig auftretende begleitende Stoffwechselstörung bei Diabetes mellitus (Zuckerkrankheit) sind und zu einer vorzeitigen Arteriosklerose führen können, ist bereits in einer Reihe von Studien eine gleichzeitige Behandlung von hyperlipidämischen Diabetikern mit Sulfonylharnstoffen bzw. Insulin und Bezafibrat untersucht worden. Der gegenüber einer alleinigen Behandlung mit Sulfonylharnstoffen bzw. Insulin eintretende Abfall der Serumtriglycerid- und -cholesterinkonzentration führt dabei auch zu einem signifikanten Absinken der Nüchternblutzuckerwerte. Alle drei Parameter steigen nach Absetzen des Bezafibrats wieder an. Es wird angenommen, daß die starke Triglyceridsenkung unter Bezafibrat, ebenso wie bei entsprechender Diät, den parallelen Rückgang der Glucose nach sich zieht. Eine Verbesserung der Glucoseutilisation durch Bezafibrat wird vermutet [vgl. Bruneder et al, Dtsch. med. Wschr. 106, (1981), 1653-1656].

Überraschenderweise wurde nun gefunden, daß auch bei nicht mit Insulinreleasern behandelten Diabetikern Typ II, bei denen die Triglycerid-, Cholesterin- und HDL-Cholesterinwerte im Normbereich liegen, so daß keine Indikation für die Anwendung von Lipidsenkern besteht, die Blutzuckerwerte jedoch trotz einer strengen Diät noch signifikant über der Norm liegen, durch Applikation von Bezafibrat eine deutliche Verbesserung der Glucosespiegel sowohl im Nüchternwert als auch nach Belastung mit einer Testmahlzeit eintritt. Auch die Triglycerid- und Cholesterinwerte sinken durch die Behandlung ab, bleiben jedoch im Normbereich.

Erfindungsgemäß wird daher vorgeschlagen - wie durch die Ansprüche näher ausgeführt - Bezafibrat auch zur Behandlung von normolipidämischen Diabetes mellitus Typ II zu verwenden. Die zu applizierende Dosis liegt vorzugsweise bei 200-600 mg/Tag. Vorteilhafterweise wird die Tagesdosis zusammen mit der Mahlzeit als Tablette oder Dragee eingenommen. Soweit ein Insulinsekretionsmangel für den Diabetes ursächlich ist, kann eine parallele Gabe von Insulinreleasern erforderlich sein und gewünschtenfalls beide Wirkstoffe in einer Dosiseinheit zusammengefaßt werden. Als Insulinreleaser kommen vorzugsweise Sulfonylharnstoffe, insbesondere Glibenclamid infrage.

Versuchsbericht

Nicht Insulin abhängige Diabetiker (NIDDM), welche nicht mit anderen Glucosespiegel-senkenden Medikamenten behandelt wurden, wurden ca. 1 Monat mit einer Diät zur Einstellung normaler Blutwerte behandelt. Danach wurden nach 10-stündigem Fasten über Nacht die Nüchternwerte (Triglycerid, Cholesterin, Glucose, Insulin) im Serum bestimmt und eine Standard-Mahlzeit mit 500 kcal verabfolgt. Anschließend wurden die Insulin- und Blutzuckerwerte in 30 Min. Abstand gemessen. Anschließend wird 3 Monate lang unter Beibehaltung der Diät mit 3x 200 mg/Tag Bezafibrat bzw. einer entsprechenden Placebotablette (in einem einfach Blindtest) therapiert und wieder Nüchternwerte und Belastungswerte nach einer erneuten Testmahlzeit gemessen. Die Ergebnisse sind in der folgenden Tabelle zusammengefaßt, wobei bei den Belastungstests jeweils der Maximalwert aufgeführt ist. In der Placebogruppe wurde weder bei den Triglycerid- noch bei den Glucosewerten ein signifikanter Unterschied zum Ausgangswert gefunden, so daß auf deren Wiedergabe verzichtet wurde.

Die Werte der Tabelle belegen, daß bei Bezafibrattherapie die Blutglucosespiegel sowohl im nüchternen Zustand, als auch unter Belastung um 10-20 % abnehmen, obwohl gleichzeitig die Insulinspiegel stark absinken. Bezafibrat erweist sich somit als brauchbares Mittel auch zur Behandlung solcher Fälle von normolipidämischen Diabetes mellitus Typ II.

Die pharmazeutische Zusammensetzung des erfindungsgemäßen Antidiabetikums ist die gleiche wie für den handelsüblichen Lipidsenker "Cedur®" oder "Bezalip®" d.h. es werden Dragees mit einem Gehalt von 200 mg Bezafibrat oder 400 mg Bezafibrat als Retardform neben üblichen Träger- und Hilfsstoffen, wie z.B. Zucker, Stärke und Cellulose, eingesetzt.

Tabelle

| | Patient 1 0 | Patient 1 3 Mon. | Patient 2 0 | Patient 2 3 Mon. | Mittelwert aus 36 Patienten 0 | Mittelwert aus 36 Patienten 3 Mon. | Norm für "Gesunde" |
|---|---|---|---|---|---|---|---|
| Triglyceride mmol/l | 2,01 | 1,45 | 1,37 | 0,65 | 2,20 | 0,40 | 0,46-1,72 |
| Cholesterin mmol/l | 7,5 | 5,7 | 7,8 | 5,9 | | | 3,9-8,5 |
| Glucose mmol/l nüchtern | 11,2 | 9,2 | 8,9 | 7,4 | 10,6 | 9,1 | 3,9-6,4 |
| belastet | 16,0 | 14,6 | 14,4 | 12,3 | 15,2 | 14,1 | |
| Insulin mU/l nüchtern | 24,9 | 13,9 | 4,0 | 3,1 | | | |
| belastet | 79,0 | 56,4 | 30,5 | 25,6 | | | |

**Patentansprüche**

1.  Verwendung von Bezafibrat für die Herstellung von Arzneimitteln zur Behandlung von Diabetes mellitus unter gleichzeitiger Senkung des Blutzucker- und Insulinspiegels.

2.  Verwendung gemäß Anspruch 1, wobei in der Dosiereinheit zusätzlich ein Insulinreleaser enthalten ist.

3

EP 0 305 890 B1

**Claims**

1. Use of bezafibrate for the preparation of medicaments for the treatment of diabetes mellitus with simultaneous sinking of the blood sugar and insulin level.

2. Use according to claim 1, whereby an insulin releaser is additionally contained in the dosage unit.

**Revendications**

1. Utilisation du bézafibrate pour la préparation de médicaments destinés au traitement du diabète sucré, avec abaissement simultané des taux sanguins de sucre et d'insuline.

2. Utilisation selon la revendication 1, dans laquelle la dose unitaire comprend également un agent insulinolibérateur.

4